# EUROPEAN PATENT SPECIFICATION

(11) **EP 3 762 054 B1**
(45) Date of publication and mention of the grant of the patent: **30.08.2023**
(21) Application number: 19721721.9
(22) Date of filing: 08.03.2019
(51) Int. Cl.: A61L 29/16, A61L 29/00, A61L 29/08, A61L 31/16

(54) **USES OF CYANOBACTERIUM EXTRACELLULAR POLYMER, COMPOSITIONS, COATED SURFACES OR ARTICLES**
VERWENDUNGEN VON EXTRAZELLULÄREM CYANOBAKTERIUMPOLYMER, ZUSAMMENSETZUNGEN, BESCHICHTETE OBERFLÄCHEN ODER ARTIKEL
UTILISATIONS DE POLYMÈRE EXTRACELLULAIRE DE CYANOBACTÉRIE, COMPOSITIONS, SURFACES REVÊTUES OU ARTICLES REVÊTUS

(30) Priority: 08.03.2018 PT 2018110614; 03.05.2018 PT 2018110718
(43) Date of publication of application: 13.01.2021
(73) Proprietor: Instituto De Biologia Molecular E Celular - IBMC, 4200-135 Porto (PT); INEB-Instituto Nacional De Engenharia Biomédica, 4200-135 Porto (PT)
(72) Inventor: GIL VASCONCELOS MOTA, Rita, 4350-417 Porto (PT); TAMAGNINI, Paula, 4450-649 Matosinhos (PT); COSTA MOUTINHO, Fabíola, 4470-139 MAIA (PT); TEIXEIRA LOPES DA COSTA PINTO, M. Cristina, 4740-532 Esposende (PT)
(74) Representative: Patentree
(86) International application number: PCT/IB2019/051904
(87) International publication number: WO 2019/171344

(56) References cited:
- WO-A1-2018/042378
- MOTA RITA ET AL: "Production and characterization of extracellular carbohydrate polymer fromCyanothecesp. CCY 0110", CARBOHYDRATE POLYMERS, APPLIED SCIENCE PUBLISHERS, LTD. BARKING, GB, vol. 92, no. 2, 3 November 2012 (2012-11-03), pages 1408-1415, XP028972831, ISSN: 0144-8617, DOI: 10.1016/J.CARBPOL.2012.10.070 cited in the application
- CAMPOCCIA DAVIDE ET AL: "A review of the biomaterials technologies for infection-resistant surfaces", BIOMATERIALS, ELSEVIER SCIENCE PUBLISHERS BV., BARKING, GB, vol. 34, no. 34, 15 August 2013 (2013-08-15), pages 8533-8554, XP028697271, ISSN: 0142-9612, DOI: 10.1016/J.BIOMATERIALS.2013.07.089 cited in the application
- BRUNA COSTA ET AL: "Broad-Spectrum Anti-Adhesive Coating Based on an Extracellular Polymer from a Marine Cyanobacterium", MARINE DRUGS, vol. 17, no. 4, 24 April 2019 (2019-04-24) , page 243, XP055601274, DOI: 10.3390/md17040243

## Description

### Technical field

The present disclosure relates the use of a polymer released from *Cyanothece* sp. CCY 0110 as an anti-adhesive or, as a preventer of bacterial adhesion or, as an anti-fouling.

The present disclosure relates to compositions comprising the polymer of the present disclosure and coated articles or surfaces.

The invention relates to a composition comprising the polymer released from Cyanothece sp. CCY 0110 deposited as CCAP number 1435/2 as a coating on a medical device for use in the preventive treatment of biofilm-mediated medical device associated infections in a subject.

The invention also relates to a coated surface or article coated, wherein the coating is on a medical device and comprises the polymer released from Cyanothece sp. CCY 0110 deposited as CCAP number 1435/2, for use in the preventive treatment of biofilm-mediated medical device-associated infections in a subject.

Further embodiments of the invention are disclosed in the dependent claims.

Wherever the terminology "embodiment" is used in the description for an aspect not falling within the scope of the claims, this terminology shall be interpreted as an embodiment of the disclosure, not being part of the invention.

### Background

Hospital-acquired infections (HAls) are considered a major health challenge in healthcare units worldwide, resuming in increased morbidity, mortality and medical costs. The prevalence rate of HAls, which are primarily caused by bacterial colonization of a broad range of biomedical surfaces, generally ranges from 4% to 10% (reaching up to 30% in intensive care units) in western-industrialized countries, making them the sixth leading cause of death (Cloutier et al., 2015). It is widely accepted that infection is frequently associated with biofilm formation. Biofilms typically consist of densely packed, bacterial populations, encased in a self-synthesized polymeric matrix, and attached to a tissue or surface where they can persist for extended periods, acting as a reservoir of pathogens and multiplying their pathways of transmission (Costerton et al., 1999). The presence of bacteria in a biofilm drastically reduces their susceptibility to antimicrobial drugs and host defense cells. Reduced susceptibility seems not to be caused by limited antibiotic penetration, but also to reduced growth rate of bacteria in a biofilm makes, which makes them less susceptible to growth-dependent antimicrobial killing. Moreover, the interstitial milieu surrounding implants is known to represent an immuno-incompetent fibro-inflammatory zone, susceptible to microbial colonization, and favorable to the instauration of infections (Salwiczek et al., 2014).

Typical treatment methods for biofilm-mediated infection of medical devices involve prolonged antibiotic therapy with possible surgical replacement of the contaminated devices, which multiplies the associated healthcare costs. In addition, the aging of global population and the rise of bacterial resistance to conventional antibiotics in clinical use should be taken into account. Anti-infective biomaterials have progressively become a primary strategy to prevent medical device-associated infections after the achieved improvements in terms of aseptic techniques, control of environment sterility and perioperative antibiotic prophylaxis. There are three major strategies for designing antibacterial coatings: (i) antibacterial agent release, mainly using antibiotics or silver compounds, which can lead to cytotoxicity, inflammatory responses and increase in resistance of bacterial strains; (ii) contact-killing, mainly using cationic compounds or enzymes; and (iii) anti-adhesion/bacteria-repelling, preventing the earliest step of biofilm formation using non-cytotoxic mechanisms (Campoccia et al., 2013).

These facts are disclosed in order to illustrate the technical problem addressed by the present disclosure.

### General Description

The present disclosure relates to a coating with anti-adhesive properties (in order to repel or prevent bacterial adhesion) based on a naturally produced polymer. This polymer that has been extensively characterized and is produced by a marine bacterium - *Cyanothece* sp. CCY 0110, at optimized production conditions to obtain increased amounts of polymer (Mota et al., 2013). Importantly, this strain is among the highly efficient polymer producers, secreting the polymer directly to the media, allowing the easy recovery of polymer with few purification steps, being a cost-effective product. The polymer has a complex composition, including nine different monosaccharides, peptides and sulfate groups, and presents thermostability up to 250ºC. Furthermore, it is non-toxic to human dermal neonatal fibroblasts (Leite et al., 2017). The overall anionic nature, hydrophobicity, and different possible conformations of this polymer make it very attractive for biotechnological applications, namely for the development of new anti-adhesive coatings.

In an embodiment, the anti-adhesive polymer of the present disclosure can be obtained from the unicellular cyanobacterium *Cyanothece* sp. CCY 0110 (Culture Collection of Yerseke, The Netherlands) grown in flasks with ASNIII medium, at 25°C under a 16 h light (30 µE m-2 s-1)/8 h dark regimen with aeration (1.2 L min-1).

The anti-adhesive polymer of the present disclosure is released from the unicellular cyanobacterium *Cyanothece* sp. CCY 0110. The cells can be oval or cylindrical and can be found in groups. *Cyanothece* sp. CCY 0110 can be purchased in the Culture Collection of Yerseke, The Netherlands. (Isolated from: Chwaka, Zanzibar. Type of sample: sediment. Incubation: 23°C. SSU RRNA SEQUENCE: max identity 99% *Cyanothece* sp. ATCC S1142, 1326 bp).

In an embodiment, the polymer coating of the present disclosure may include a polydopamine (pDA) layer, which allows ultrathin homogenous film of the polymer linkage to the surface of targeted materials. pDA has excellent biocompatibility, having the ability to be applied to different materials surfaces (polymers, metals, ceramics) acting as a "primer" for further surface modifications.

In an embodiment, different substrates were used: (i) materials frequently used for medical devices production (i.e. polyurethane (PU) and silicone (Si) substrates) and (ii) gold substrates, due to its higher suitability for the surface characterization techniques.

An aspect of the present disclosure is the use of a polymer released from *Cyanothece* sp. CCY 0110 as an anti-adhesive or, as a preventer of bacterial adhesion or, as an anti-fouling. This polymer has shown unique characteristics as an anti-adhesive coating with high potential to prevent medical device-associated infections. The anti-adhesive properties of the polymer released from *Cyanothece* sp. CCY 0110 of the present disclosure are maintained even after 30 days of storage, in standard conditions (shelf-life).

The present disclosure also relates to a composition comprising a polymer released from *Cyanothece* sp. CCY 0110. In particular an anti-fouling paint or an anti-adhesive composition.

In an embodiment for better results, the composition may further comprise at least one of the following components: a dye, a paint, a varnish, a polish, a primer, or mixtures thereof.

In an embodiment the composition may comprise 0.01-2% (w/v) of polymer; preferably 0.05-1% (w/v) of polymer; more preferably 0.1-0.5% (w/v) of polymer.

In an embodiment for better results, the primer is polydopamine, among other suitable primers or surface activators.

Another aspect of the present disclosure relates to a coated surface or article comprising the polymer released from *Cyanothece* sp. CCY 0110 or the composition according to any of the previous embodiments.

In an embodiment for better results, the surface is a metal, a polymer, a plastic, a ceramic. Preferably the surface is polyurethane, silicone, titanium, medical steel, gold, hydroxyapatite, dental/orthodontic cement, orthodontic device or mixtures thereof.

In an embodiment for better results, the article is a medical device. In particular, a catheter, a syringe, a stent, a tube, a medical packaging, a dialysis device, a dental prosthesis, among others.

Anti-adhesive properties of the polymer released from *Cyanothece* sp. CCY 0110 of the present disclosure are maintained even in the presence of blood plasma proteins.

The polymer of the present disclosure reduces adhesion (87%) and activation (90%) of platelets comparing to reference material, polyurethane (PU), even in the presence of blood plasma proteins.

The polymer of the present disclosure is stable in accelerated degradation conditions (7 days, 45°C, pH7.4 & pH5), according to ISO 10993-13:2009 (Biological evaluation of medical devices - Part 13: Identification and quantification of degradation products from polymeric medical devices).

Also, the polymer released from *Cyanothece* sp. CCY 0110 of the present disclosure has no cytotoxic potential according to ISO 10993-5:2009(E) (Biological evaluation of medical devices - Part 5: Tests for in vitro cytotoxicity).

An aspect of the present disclosure relates to a composition comprising the polymer released from *Cyanothece* sp. CCY 0110 as a coating on a medical device for use in the preventive treatment of biofilm-mediated medical device associated infection in a subject, namely in the preventive treatment catheter-associated infections (CAI) or biofilm-mediated medical device associated infection in a subject.

In an embodiment, the catheter-associated infection (CAI) or biofilm-mediated medical device associated infection in a subjectis caused by *S. epidermidis or S. aureus.*

In an embodiment, the catheter-associated infection (CAI) or biofilm-mediated medical device associated infection in a subject is caused by *S*. *epidermidis.*

In an embodiment, the catheter-associated infection (CAI) or biofilm-mediated medical device associated infection is caused by a highly adhesive *S*. *epidermidis* strain in the presence of human plasma proteins.

In an embodiment, said composition is further used for preventing platelet adhesion to the medical device and activation of said platelets (anti-thrombogenic effect).

Another aspect of the present disclosure relates to a coated surface or article coated comprising the polymer released from *Cyanothece* sp. CCY 0110.

Another aspect of the present disclosure relates to the coated surface or article wherein the catheter-associated infection (CAI) or biofilm-mediated medical device associated infection in a subject is caused by *S*. *epidermidis* or *S. aureus.*

Another aspect of the present disclosure relates to the coated surface or article wherein the catheter-associated infection (CAI) or biofilm-mediated medical device associated infection in a subject is caused by a highly adhesive *S*. *epidermidis* strain in the presence of human plasma proteins.

In an embodiment, said coated surface or article is further used for preventing platelet adhesion to the medical device and activation of said platelets (anti-thrombogenic effect).

Another aspect of the present disclosure relates to a coated surface or article coated comprising the polymer released from *Cyanothece* sp. CCY 0110 for use in preventive treatment of catheter-associated infection (CAI) or biofilm-mediated medical device associated infection in a subject.

In an embodiment, the composition or coated surface or article further comprises at least one of the following components: a dye, a paint, a varnish, a polish, a primer, a surface activator, a coating or mixtures thereof.

In an embodiment, the composition or the coated surface or article comprises at least 0.01% (w/v) of the polymer.

In an embodiment, the composition or the coated surface or article comprises at least 0.05% (w/v) of the polymer.

In an embodiment, the composition or the coated surface or article comprises at least 5% (w/v) of the polymer.

In an embodiment, the composition or the coated surface or article comprises at least 10% (w/v) of the polymer.

In an embodiment, the composition or the coated surface or article comprises at least 50% (w/v) of the polymer.

In an embodiment, the composition or the coated surface or article comprises 0.01-2% (w/v) of polymer; preferably 0.05-1% (w/v) of polymer; more preferably 0.1-0.5% (w/v) of polymer.

In an embodiment, the composition or the coated surface or article is a medical device, preferably selected from the list consisting of a catheter, a syringe, a stent, a tube, a medical packaging, a dialysis device, a dental prothesis, dental implant, and an orthodontic device.

Another aspect of the present disclosure relates to a coated surface or article coated with the polymer released from *Cyanothece* sp. CCY 0110, for use in the preventive treatment of biofilm-mediated medical device associated infections in a subject, wherein said coated surface or article is implanted in an animal or human body.

In an embodiment, the coated surface or article further comprises at least one of the following components: a dye, a paint, a varnish, a polish, a primer, a surface activator, a coating or mixtures thereof.

In an embodiment, the coated surface or article for use in preventing platelet adhesion to the coated surface or coated article and activation of said platelets comprises at least 0.01% (w/v) of the polymer.

In an embodiment, the coated surface or article for use in preventing platelet adhesion to the coated surface or coated article and activation of said platelets comprises at least 0.05% (w/v) of the polymer.

In an embodiment, the coated surface or article for use in preventing platelet adhesion to the coated surface or coated article and activation of said platelets comprises at least 5% (w/v) of the polymer.

In an embodiment, the coated surface or article for use in preventing platelet adhesion to the coated surface or coated article and activation of said platelets comprises at least 10% (w/v) of the polymer.

In an embodiment, the coated surface or article for use in preventing platelet adhesion to the coated surface or coated article and activation of said platelets comprises at least 50% (w/v) of the polymer.

In an embodiment, the coated surface or article for use in preventing platelet adhesion to the coated surface or coated article and activation of said platelets comprises 0.01-2% (w/v) of polymer; preferably 0.05-1% (w/v) of polymer; more preferably 0.1-0.5% (w/v) of polymer.

In an embodiment, the coated surface or article is a medical device, preferably selected from the list consisting of a catheter, a syringe, a stent, a tube, a medical packaging, a dialysis device, a dental prothesis, dental implant, and an orthodontic device.

### Brief Description of the Drawings

The following figures provide preferred embodiments for illustrating the disclosure and should not be seen as limiting the scope of invention.
**Figure 1****.** Polymer coating of the present disclosure surface characterization. A) thickness measurements; B) water contact angle measurements.
**Figure 2****.** Polymer coating of the present disclosure surface characterization before and after 30 days of storage. A) thickness measurements; B) water contact angle measurements (*p<0.05, non-parametric Kruskal-Wallis).
**Figure 3****.** Anti-adhesive performance of the polymer coating of the present disclosure according to ISO 22196 against A) *Staphylococcus epidermidis*; B) *S*. *aureus*; C) *Escherichia coli* (****p<0.0001, n.s.- non-significant; non-parametric Kruskal-Wallis).
**Figure 4****.** Anti-adhesive performance of the polymer coating of the present disclosure in the absence and presence of human plasma proteins 1% (v/v) against S. epidermidis according to ISO 22196 (**p<0.008; *p<0.03; non-parametric Kruskal-Wallis).
**Figure 5****.** Biocompatibility assessment of the polymer coating of the present disclosure according to ISO 10993-5:2009(E): Relative metabolic activity (measured by MTT) of L929 fibroblasts after incubation with polymer coating of the present disclosure extracts prepared according to ISO 10993-12:2004 (results presented as % of positive control L929).
**Figure 6****.** Representative scanning electron microscopy (SEM) micrographs of platelets adhesion to the gold, PU and polymer coating of the present disclosure in the presence of 1% (v/v) human plasma proteins (1000× magnification, scale 60 µm).
**Figure 7****.** Platelet adhesion and activation profile on top of the polymer coating of the present disclosure characterization using SEM analysis, according to ISO 10993-4:2009. A) without plasma proteins; B) with 1% (v/v) plasma proteins.
**Figure 8****.** Degradation assay of the polymer coating of the present disclosure performed according to ISO 10993-13:2009 (45°C, pH7.4, pH5). A) polymer coating thickness; B) polymer coating water contact angle measurements; C) polymer coating anti-adhesive performance against S. epidermidis (*p<0.05; ****p<0.001 non-parametric Kruskal-Wallis).
**Figure 9****.** Representative SEM micrographs of platelets adhered to PU-coating categorized by activation state: (A) Non-activated, (B) Partially activated and (C) Fully activated (magnification: A- 15 000×; B- 10 000×; C- 10 000 ×).
**Figure 10****.** Number of adhered platelets to PU and polymer per µm2 after 30 min of incubation at 37 °C in presence or absence of plasma proteins (1%, v/v) (n = 9). Statistical analysis was performed by Non-parametric Kruskal Wallis analysis and differences are indicated with **** (p < 0.001).

### Detailed Description

In an embodiment, the polymer is obtained from the unicellular marine cyanobacterium *Cyanothece* sp. CCY 0110 (Culture Collection of Yerseke, the Netherlands) grown in bioreactors (Stirred reactors, DWK Life Sciences) with ASNIII medium (Rippka et al., 1979) at 25°C under a 16 h light (30 µE m-2 s-1)/8 h dark regimen with aeration (1.2 L.min-1) and orbital stirring (150 rpm) (Mota et al., 2013) until an OD730nm of ≈ 3.0 - 3.5.

For the isolation of the released polymer the cultures are placed in dialysis membranes (12-14 kDa of molecular weight cut-off; Medicell, UK) and dialyzed against a minimum of ten volumes of type II water for 48 h in continuous stirring. Cells are removed by centrifugation at 20.000 × g for 15 min at 4°C. The polymer (in the supernatant) is precipitated with two volumes of 99% cold ethanol. The polymer is collected with sterile metal forceps, dissolved in type II water, and precipitated again as previously described and lyophilized.

In an embodiment, the pyrogenic/endotoxin effects assessment was performed by serially diluting aqueous solutions of the polymer 1.0% (w/v), sterilize them through 0.2 µm filters and incubate them with whole fresh blood overnight. Plasma was isolated and analyzed by enzyme-linked immunosorbent assay (ELISA) for cytokine interleukin-1β (IL-1β) release. Test wells were compared to endotoxin treated controls to confirm no or low pyrogenic activity (target < 0.5 EU/mL Etaxate) (Daneshian et al., 2009).

In an embodiment, the gold substrate preparation was performed according to Martins et al., 2003. Briefly, chromium (5 nm) and gold (25 nm) layers were deposited by ion beam sputtering from chromium and gold targets (99.9% purity) on silicon wafers (AUREL, GmbH). Chromium was used to improve the adhesion of gold to silicon. Gold substrates (1×1cm) were cleaned with "piranha" solution (7 parts of H2SO4 and 3 parts of 30% H2O2) for 5 min, thoroughly rinsed with ethanol and dried with a gentle stream of argon.

In an embodiment, a polyurethane (PU) film was produced from PU pellets (Pellethane 2363-80 AE, Dow Chemical). PU pellets were initially sonicated (15 min) twice with hexane (Merck) and once with ethanol (99.9%, Merck) to eliminate silicone casing. This process was repeated twice (Felgueiras et al., 2017). Finally, PU pellets were rinsed with type II water and left to dry overnight in a vacuum oven at room temperature (RT) for 24 h. PU pellets were dissolved in tetrahydrofuran (THF, Sigma) at 0.1% (w/v). PU thin films were prepared by spin coating PU solution on top of gold substrate (Laurell Technologies Corporation) at 9000 rpm for 1 min. PU films were dried in a vacuum oven at RT for 1 h.

In an embodiment, a polydopamine (pDA) layer was introduced to the samples in order to promote the linkage of polymer of the present disclosure to the gold substrate. Dopamine hydrochloride (H8502, Sigma-Aldrich) solution was freshly prepared in Tris-HCl 10 mM pH8.5 buffer, in different concentrations (0.1 mg/mL, 1 mg/mL and 2 mg/mL). Gold substrates were placed in a 24-well suspension cells plate (Starsted), immersed in 1 mL of dopamine solution and incubated for 2 h (1 mg/mL and 2 mg/mL), 18 h (0.1 mg/mL) or 24 h (2 mg/mL) at RT and 70 rpm, protected from light. Samples were rinsed twice with Tris-HCl 10 mM pH8.5 buffer and once with type I water, using an ultrasound bath (1 min). pDA-coated samples were stored no more than 2 h after use.

In an embodiment, for the production of the polymer solution, the polymer of the present disclosure was hydrated in type II water (0.5% w/v) for 24h. Then, the polymer solution was autoclaved at 110ºC for 30 min. After cooling, polymer solution was spin coated on top pDA-coated gold substrates at 9000 rpm for 1 min (Laurell Technologies Corporation). Polymer coatings were dried in a vacuum oven at room temperature for 1 h.

In an embodiment, thickness measurements were performed using an imaging ellipsometer, model EP3, from Nanofilm Surface Analysis. This ellipsometer was operated in a polarizer-compensator-sample-analyzer mode (null ellipsometry). The light source was a solid-state laser with a wavelength of 532 nm. The gold substrate refractive index (n = 0.709) and extinction coefficient (k = 2.534) were determined using a delta and psi spectrum with a variation of angle between 60 and 81°. These measurements were made in four zones to correct for any instrument misalignment. The thickness of the pDA layer and polymer coating was determined using (n) polymer = 1.54 and (k) polymer = 0.

In an embodiment, contact angle measurements were performed (Water contact angle (WCA)) using the inverted drop method (captive air-bubble method) with Data Physics, model optical contact angle (OCA) 15, equipped with a video CCD camera and SCA 20 software. Each sample was tape-glued to a glass slide and placed into a type II water-filled glass chamber. After 6 µL bubble of air release from a J-shaped needle placed underneath the sample surface, images were taken, and air-bubble profile was fitted using different mathematical functions, to calculate the contact angle. The Laplace-Young fitting method was used to calculate contact angles >60° and the tangent fitting method was used to calculate contact angles <60°.

In an embodiment, bacterial strains, media and growth conditions were obtained. In particular, *Staphylococcus epidermidis* (ATCC 35984), *S. aureus* (ATCC 33591) and *Escherichia coli* (ATCC 25922) were obtained from the American Type Culture Collection. Bacteria were grown on tryptic soya agar (TSA) (Merck) and tryptic soya broth (TSB) (Merck). Bacterial suspensions were adjusted by measuring optical density (600 nm). Bacterial numbers were confirmed by a colony forming units (CFUs) count.

In an embodiment, bacterial-polymer coating interaction was measured. In particular, in order to assess surface resistance against bacterial adhesion, ISO 22196 (Measurement of antibacterial activity on plastics and other non-porous surfaces) was applied. Firstly, samples were disinfected twice with ethanol 70% for 15 min and rinsed twice sterilized type II water for 15 min. Finally, samples were dried with a gentle stream of argon. Then, samples were challenged with 5 µL of bacterial suspension at 1.8x106 CFUs/mL (with and/or without 1% (v/v) human plasma proteins) in TSB. Polypropylene (PP) coverslips (∅=9mm) were applied on top bacterial suspension drop to force bacteria contact with the surface. After 24 h incubation at 37ºC high humidity, samples were rinsed with PBS. Adhered bacteria were fixed with paraformaldehyde 4% and stained with 4',6-diamidino-2-phenylindole (DAPI). Inverted fluorescence microscopy (IFM) was used to visualize adherent bacteria in the DAPI channel (excitation: 365 nm; emission: 420 nm) and acquire images. The number of adherent bacteria was quantified using the Image J software.

In an embodiment, the biocompatibility of the polymer coating of the present disclosure was evaluated by the incubation of mouse fibroblasts L929 with extracts of polymer coating as described in ISO 10993-5:2009(E). The extracts were prepared as described in ISO 10993-12:2004 (Biological evaluation of medical devices - Part 12: Sample preparation and reference materials). Briefly, polymer coatings were sterilized as explained in [0071]. After the sterilization step, polymer coatings were rinsed with PBS and extracted with MEM culture medium (Modified Eagle's medium (MEM, Gibco)) supplemented with 10% (v/v) Fetal bovine serum (Gibco) and 1% (v/v) penicillin/streptomycin (Biowest) at 37ºC with a humified atmosphere of 5% CO2 for 24 h. Cells were seeded in 96-well plates at density of 1×105 cells/mL and maintained in culture for 24 h in supplemented MEM. After that, the medium was replaced by material extracts and respective 50% dilutions, according to ISO 10993-5:2009(E). After 24 h of incubation at 37ºC with a humified atmosphere of 5% CO2, metabolic activity of cells was quantified by MTT assay. Extracts of clean wells were used as positive control of cells growth while a solution of MEM with 50% DMSO was used as negative control.

In an embodiment, to assess the thrombogenicity of the polymer coatings of the present disclosure ISO 10993-4:2009 (Biological evaluation of medical devices -Part 4: Selection of tests for interactions with blood) was applied. Platelets from an Intermediary Platelet Unit (IPU), provided by Hospital de São João, Porto, were used. Au, PU, and polymer coatings samples were sterilized as described in [0071]. After sterilization, samples were immersed in 1% (v/v) plasma in PBS, for 30 min at 37°C, and rinsed 3 times with PBS. Simultaneously, 24-well tissue culture polystyrene plates (TCPS, Sarstedt) were incubated at with 1% (w/v) bovine serum albumin (BSA) in PBS for 1 h, to reduce platelet activation in response to the Tissue culture-treated polystyrene (TCPS). Protein-adsorbed surfaces were transferred to the BSA-treated plates, previously washed 5 times with PBS, and incubated with IPU at 3×108 platelets/mL in PBS for 30 min at and 70 rpm. Surfaces were rinsed with PBS and the adhered platelets fixated with 1.5% (v/v) glutaraldehyde (Merck) in 0.14 M sodium cacodylate (Merck) buffer for 30 min at RT. Finally, platelet-adhered surfaces were dehydrated in a growing ethanol/water gradient, 50, 60, 70, 80, 90 and 99% (v/v), for 10 min each. To avoid superficial tension effects and preserve the materials and the adhered platelets structural integrity during SEM visualization, we applied critical point drying. This process includes a series of temperature variations from 4°C to 33-38°C until a maximum pressure of 1000-1400 psi is reached. The samples conductivity was enhanced by sputtering with Au/Pd for 60 s and 15 mA current. Five micrographs of each film were taken by SEM using an electron beam intensity of 5 kV and magnification of 2000x. The number of platelets per surface condition was determined using the Image J software and reported as platelets/mm2. Adhered platelets were divided per degree of activation in non-activated, partially activated and fully activated, according to a pre-established scale, and reported as percentage per degree of activation.

In an embodiment, to assess the shelf stability of the polymer coating of the present disclosure, samples produced in different time points were characterized simultaneously (as explained in [0068], [0069] and [0071]), to assess possible deterioration during storage. For the polymer coating of the present disclosure degradation susceptibility evaluation ISO 10993-13:2009 (Biological evaluation of medical devices - Part 13: Identification and quantification of degradation products from polymeric medical devices) was used. Briefly, samples were incubated in buffers with different pH (5 and 7.4) at 45°C for 7 days. At the end of the incubation periods, the samples surface was characterized with techniques described in [0068] and [0069], and was challenged with bacteria as described in [0071].

In an embodiment, the extracellular polymer produced by the marine cyanobacterium *Cyanothece* sp. CCY 0110 was harvested when the culture reached an OD730nm between 3.0 to 3.5, with a yield of approximately 2 g of polymer per g of dry weight of culture. The polymer was then tested for endotoxin-pyrogenic potential using a whole fresh blood interleukin-1β (IL-1β) release assay. Results revealed absence of endotoxin-pyrogenic effect (0 pg/mL).

In an embodiment, to identify the optimum polymer concentration of the present disclosure to be used in the coating to achieve the desired anti-adhesive characteristics, a range of polymer concentration was tested (from 0.1% to 1.0%). Results demonstrated that the concentration of polymer (0.5% (w/v)) results in a greater anti-adhesive effect of the coating. Further coating development was done using this concentration.

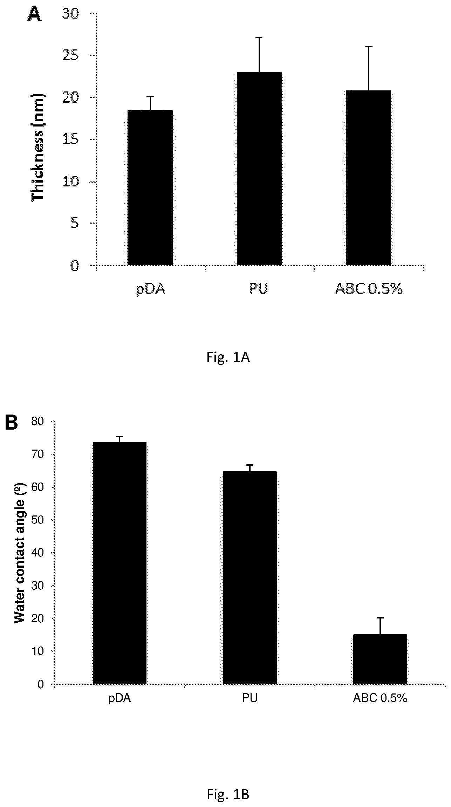

In an embodiment, the surface characterization of modified surfaces comprising the polymer of the present disclosure were analyzed by ellipsometry and water contact angle measurements (Figure 1).

In an embodiment, the thickness measurements by ellipsometry revealed that dopamine immersion resulted on a layer of 18.4 ± 1.7 nm. The spin coating process resulted on a polyurethane (PU) (0.1% (v/v)) ultrathin film of 23.0 ± 4 nm and in the polymer coating of the present disclosure with 20.7 ± 5.4 nm of thickness (Figure 1 A).

In an embodiment, water optical contact angles of the PU and polymer coating of the present disclosure production steps are presented on Figure 1 B. pDA layer has a hydrophobic profile, revealing a WCA of 73 ± 2º, PU ultrathin films presented a lower WCA of about 64.6 ± 2º. Polymer application significantly increased the hydrophilicity of the polymer coating of the present disclosure comparing both to PU and pDA layer, decreasing the WCA to 15 ± 5º.

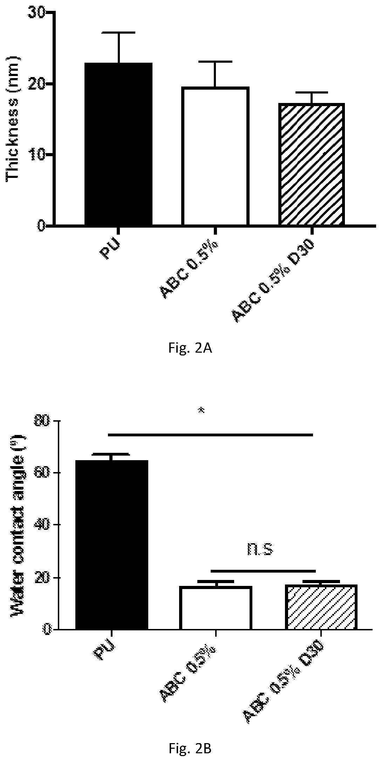

In an embodiment, to assess shelf-life stability of the polymer coating of the present disclosure, coatings were maintained under standard storage conditions (room temperature and low humidity) and then re-characterized (Figure 2).

In an embodiment, as depicted in Figure 2 A & B, no significant thickness and water contact angle differences were found on the polymer coating of the present disclosure after the 30 days storage period.

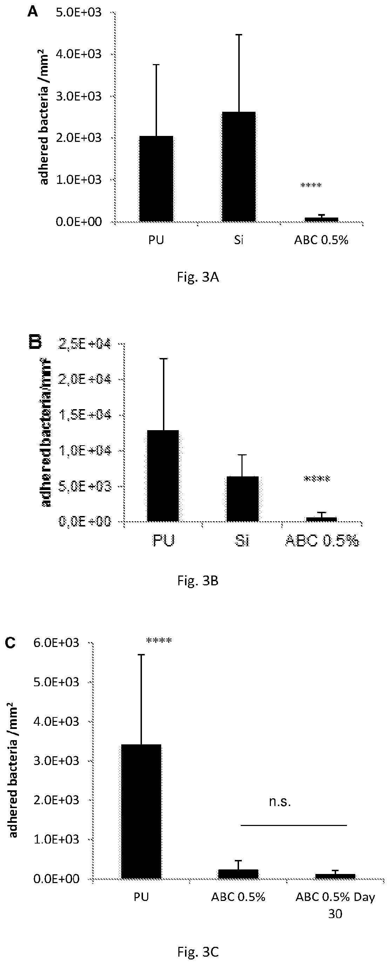

In an embodiment, the anti-adhesive activity of the polymer coating of the present disclosure was assessed according to ISO 22196 against different bacterial strains, 2 Gram-positive (*Staphylococcus epidermidis* & *S. aureus*) and 1 Gram-negative (*Escherichia coli*)*.* Results are presented in Figure 3.

In an embodiment, bacterial adhesion on the polymer released from *Cyanothece* sp. CCY 0110 of the present disclosure was always 90% lower comparing to medical devices reference materials PU and Si (Figure 3 A, B, C). Highest anti-adhesive efficacy (>95% bacterial adhesion reduction) was found against both Gram-positive (*S*. *epidermidis* and *S*. *aureus*) (Figure 3 A & B). Similar anti-adhesive efficacy was found between fresh prepared coatings comprising the polymer of the present disclosure and coatings comprising the polymer of the present disclosure after 30 days storage against *S*. *epidermidis* (Figure 3 C), meaning that the polymer coating was able to maintain activity through 30 days self-life.

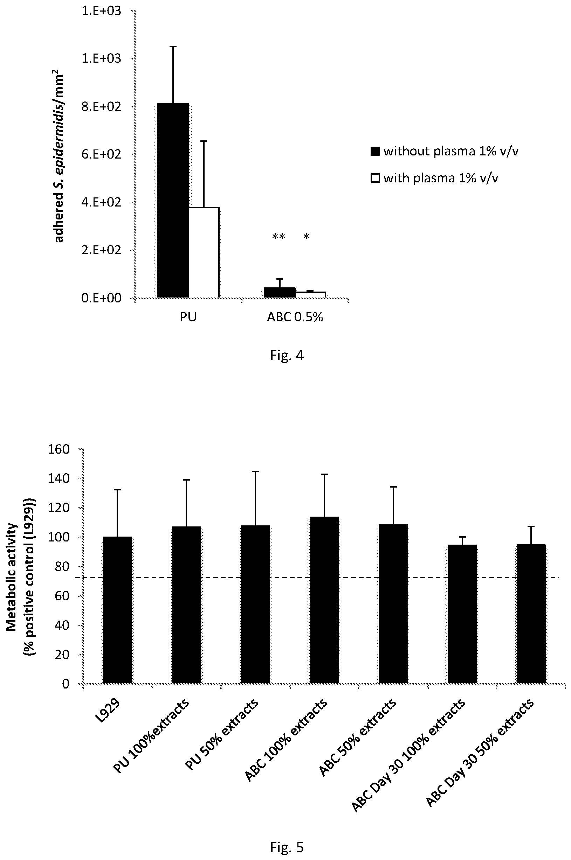

In an embodiment, in order to further challenge the polymer coating of the present disclosure with an in vitro model closer to in vivo conditions, the above assay was performed in the absence and presence of human plasma proteins 1% (v/v), using a highly adhesive *S. epidermidis* strain. Results are depicted in Figure 4.

In an embodiment, *S. epidermidis* adhesion to both surfaces was lower in the presence of human plasma proteins. Nevertheless, independently of the formation of a conditioning film polymer coating of the present disclosure was capable of promoting a 94% bacterial reduction regarding PU control surface (Figure 4).

In an embodiment, the biocompatibility of the polymer released from *Cyanothece* sp. CCY 0110 coating of the present disclosure was evaluated by the metabolic activity of mouse fibroblasts L929 after incubation with extracts of materials as described in ISO 10993-5:2009(E). The extracts were prepared as described in ISO 10993-12:2004. Results are presented in Figure 5.

In an embodiment, the metabolic activity differential between fresh prepared polymer coating of the present disclosure coating, 30 days storage polymer coating of the present disclosure and PU control was never superior to 6%, much lower than the 30% limit, demonstrating that polymer coatings surfaces have no cytotoxic potential, according to ISO 10993-5:2009(E).

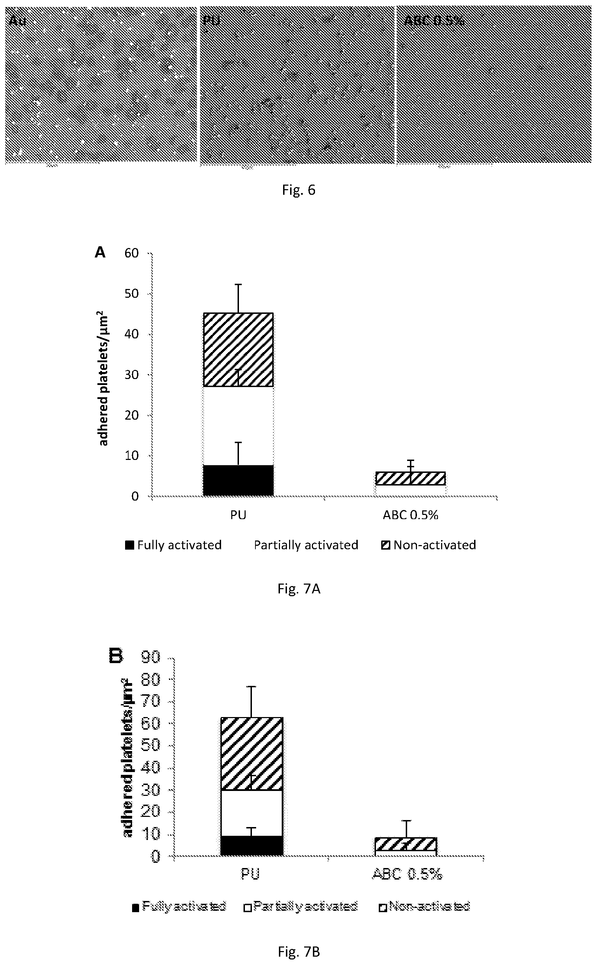

In an embodiment, the effect of the polymer released from *Cyanothece* sp. CCY 0110 coating of the present disclosure on the adhesion and activation of platelets was followed by SEM. Figure 6 depicts representative images of the amount and activation status of adhered platelets. Figure 7 shows the quantitative distribution of platelets on the surfaces according to their activation state.

In an embodiment, as reported by Figures 6 & 7 the polymer coating of the present disclosure does not promote platelet adherence, reducing in 87% the amount of adhered platelets compared to a naked surface of polyurethane (PU), even in the presence of blood plasma proteins (Figure 7 A & B). Moreover, the polymer coating of the present disclosure also prevents activation of platelets (less 90% of activated platelets regarding PU control surface).

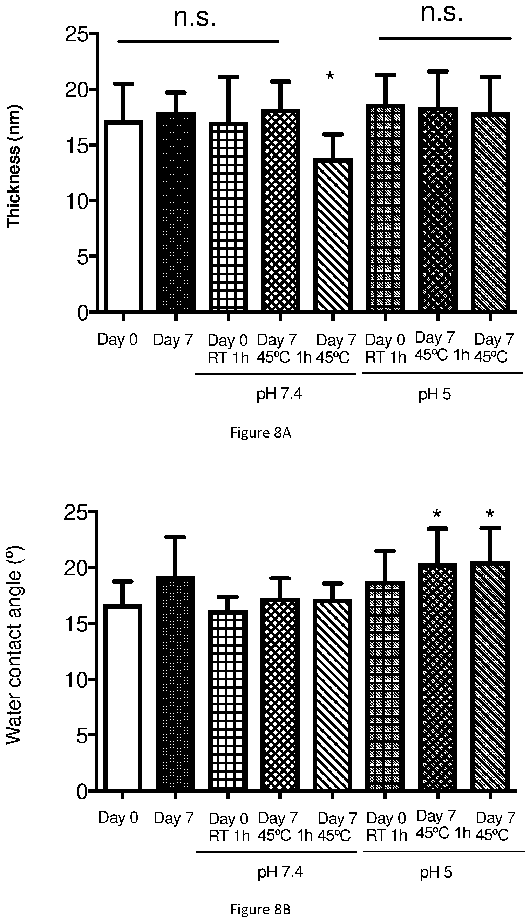

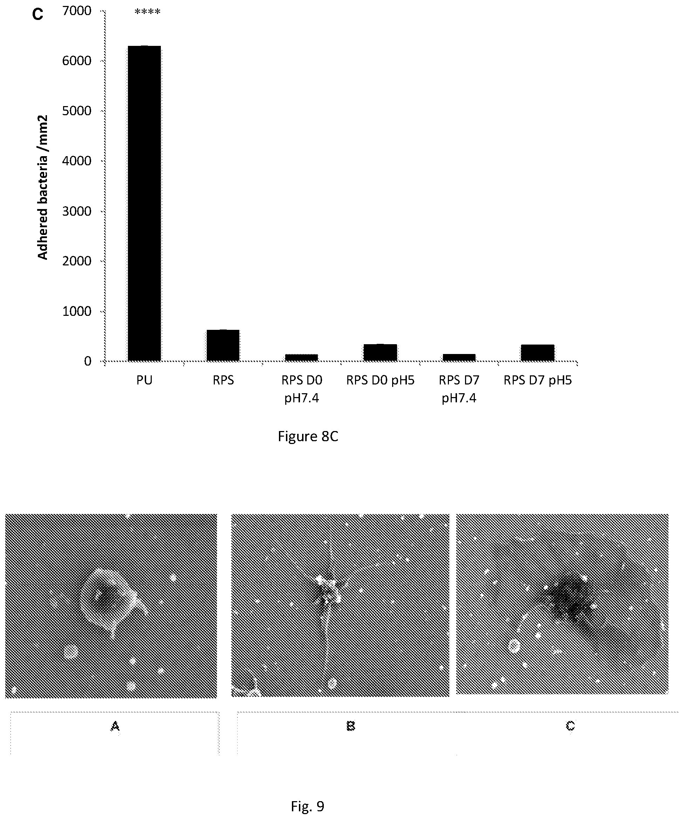

In an embodiment, the polymer coating of the present disclosure presented minor alterations at the surface after the accelerated degradation assay at both pHs (Figure 8 A & B), which did not influence its anti-adhesive performance (Figure 8 C), suggesting that the coating remained stable throughout the assay.

Where singular forms of elements or features are used in the specification of the claims, the plural form is also included, and vice versa, if not specifically excluded. For example, the term "a polymer" or "the polymer" also includes the plural forms "polymers" or "the polymers," and vice versa. In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all the group members.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

In an embodiment, the cyanobacterium Cyanothece sp. CCY 0110 used was received on 13 March 2018 and tested as viable on 26 April 2018, by the Scottish Association for Marine Science Culture Collection of Algae and Protozoa (CCAP) - International Depositary Authority under the Budapest Treaty - and given the CCAP number 1435/2.

The following examples are for illustrative purposes only and should not limit the present invention.

### EXAMPLES

### Example 1. Characterization of the polymer produced by Cyanothece sp. CCY 0110

The polymer produced by Cyanothece sp. CCY 0110 is known from Mota et al., 2013 and it is composed by nine different monosaccharides, neutral sugars 54.8±8.5% polymer dry weight (w/w), including mannose, glucose, galactose, xylose, arabinose, rhamnose and fucose, and acidic sugars, 7.4±0.8% polymer dry weight (w/w), including galacturonic and glucuronic acids; peptides 4.0±0.3% polymer dry weight (w/w) and sulfate groups 10.6±1.6% polymer dry weight (w/w).

The composition of the polymer produced by *Cyanothece* sp. CCY 0110 differs from the composition of other polymers produced from other cyanobacteria such as *Cyanothece* sp. VI 22, as it can be observed by comparing their molar ratios with respect to the rhamnose content (*Cyanothece* sp. CCY 0110/ *Cyanothece* sp. VI 22): mannose (2.14/1.0), glucose (2.45/2.75), galactose (0.88/0.25), xylose (1.30/1.79), arabinose (0.45/0), rhamnose (1.0/1.0), fucose (0.29/1.75), galacturonic acid (0.3/0) and glucuronic acid (0.28/+: present but not quantified, reported by De Philippis et al., 1998).

### Example 2. Preparation of the polymer produced by Cyanothece sp. CCY 0110

A full explanation of the preparation of the polymer produced by *Cyanothece* sp. CCY 0110 is herein provided in the detailed description of the disclosure.

At any rate, briefly, the unicellular cyanobacterium *Cyanothece* sp. CCY 0110 (Culture Collection of Yerseke, The Netherlands, now available at Culture Collection of Algae and Protozoa (CCAP 1435/2) was grown in 2 L bioreactors with ASNIII medium, at 25 °C, under a 16 h light (30 µE/m2/s)/8 h dark regimen, with orbital stirring (150 rpm) and with aeration (1.2 L/min) (Mota et al., 2013), until an optical density of ≈2.5-3.5 at 730 nm. Grown culture was placed in dialysis membranes (12-14 kDa cut-off) and dialyzed against a minimum of ten volumes of type II water for 48 h in continuous stirring. Cells were removed by high speed centrifugation (20000 g, 15 min, 4 ºC) and the supernatant was precipitated with two volumes of 99% ethanol (AGA) at 4 ºC overnight. The biopolymer was collected with sterile metal forceps, dissolved in type II water, and precipitated once more. The collected biopolymer was lyophilized, grounded (mill A10 basic, IKA) and stored in a desiccator until further steps.

### Example 3. Coating a medical device with the polymer produced by Cyanothece sp. CCY 0110

To apply the polymer coating, produced by *Cyanothece* sp. CCY 0110, to medical devices a number of different surface activation methodologies may by applied such as chemical etching, UV activation and Plasma activation. As example, an industrial atmospheric plasma system, with an estimated maximum power of 15 kW, can be used for the surface activation of polyurethane substrates. Substrates are then exposed simultaneously to an argon flow rate of 15 L/min and N2 flow rate of 0.45 L/min (11.25 kW, 3.3 Hz). After proper surface activation, the polymer solution may be applied on top through dip-coating, spray coating, flow coating or back coating. Considering dip-coating, freshly activated polyurethane substrates are immersed in 0.5% w/v polymer solution, retrieved slowly and air-dried in clean facilities.

### Example 4. The polymer produced by Cyanothece sp. CCY 0110 impedes platelet adhesion and activation assessment (Anti-thrombogenic effect)

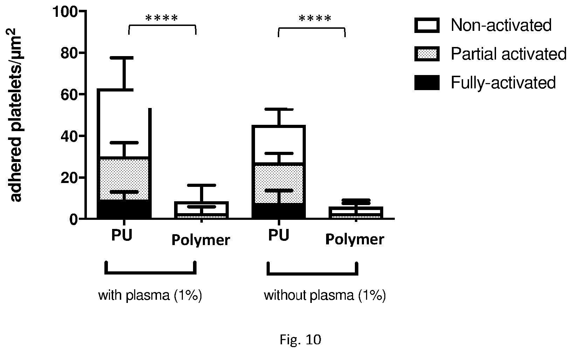

Platelets adhered to surfaces can have different activation states that affects their probability to thrombus formation. Therefore, in this example, the effect of the polymer produced by *Cyanothece* sp. CCY 0110 on the adhesion and activation of platelets (thrombogenic effect) was followed by Scanning Electron Microscopy (SEM). Three different activation states were considered as depicted in Figure 9. Results from the quantitative distribution of platelets according to their activation state in Polymer and PU are presented in Figure 10.

The results obtained demonstrate that the polymer produced by *Cyanothece* sp. CCY 0110 reduces platelet adhesion to less than 87% in comparison to PU, even in the presence of plasma proteins (1%, v/v). Indeed, in the presence of plasma proteins the number of adhered platelets augmented significantly to PU (31%), but not to Polymer whose anti-adhesive profile was maintained at 87% adhesion reduction. Importantly, the polymer produced by *Cyanothece* sp. CCY 0110 did not promote activation of platelets, since most of the adhered platelets were in low activation state (non-activated or partially activated state, but none at fully activated morphology). In opposite, platelets adhered to PU presented a higher number of partially or fully activated morphologies.

The disclosure should not be seen in any way restricted to the embodiments described and a person with ordinary skill in the art will foresee many possibilities to modifications thereof, which can only pertain to the invention if they fall within the scope of the appended claims.

The above described embodiments are combinable.

The following claims further set out particular embodiments of the invention.

### References:

Campoccia D, Montanaro L, Arciola CR (2013) A Review of the Biomaterials Technologies for Infection-Resistant Surfaces. Biomaterials 34 (34):8533-8554.
Cloutier M, Mantovani D, Rosei F (2015) Antibacterial Coatings: Challenges, Perspectives, and Opportunities. Trends Biotechnol 33(11):637-652.
Costerton JW, Stewart PS, Greenberg EP. (1999) Bacterial biofilms: a common cause of persistent infections. Science 284(5418):1318-1322.
Danieshan M, Von Aulock S, Hartung T. (2009) Assessment of pyrogenic contamination with validated human whole-blood assay. Nature Protocols 4(12):1709-1721.
De Philippis R, Margheri MC, Materassi R, Vincenzini M (1998) Potential of Unicellular Cyanobacteria from Saline Environments as Exopolysaccharide Producers. Appl Environ Microbiol 64(3):1130-1132.
Felgueiras HP, Wang LM, Ren KF, Querido MM, Jin Q, Barbosa MA, Ji J, Martins MCL (2017) Octadecyl Chains Immobilized onto Hyaluronic Acid Coatings by Thiol-Ene "Click Chemistry" Increase the Surface Antimicrobial Properties and Prevent Platelet Adhesion and Activation to Polyurethane. ACS Appl Mater Interfaces 9(9):7979-7989.
Leite JP, Mota R, Durão J, Neves S, Barrias C, Tamagnini P, Gales L (2017) Cyanobacterium-derived extracellular carbohydrate polymer for the controlled delivery of functional proteins. Macromol. Biosci. 17,1600206.
Martins MC, Ratner BD, Barbosa MA. (2003) Protein adsorption on mixtures of hydroxyl- and methyl-terminated alkanethiols self-assembled monolayers. J Biomed Mater Res A 67:158-171.
Mota R, Guimaraes R, Buttel Z, Rossi F, Colica G, Silva CJ, Santos C, Gales L, Zille A, De Philippis R, Pereira SB, Tamagnini P (2013) Production and characterization of extracellular carbohydrate polymer from Cyanothece sp. CCY 0110. Carbohydr Polym 92:1408-1415. Rippka R, Deruelles J, Waterbury JB, Herdman M, Stanier RY. (1979) J. Gen. Microbiol 111:1-61.
Salwiczek M, Qu Y, Gardiner J, Strugnell RA, LithgowT, McLean KM, Thissen H (2014) Emerging rules for effective antimicrobial coatings. Trends Biotechnol 32:82-901.

## Claims

1. A composition comprising the polymer released from Cyanothece sp. CCY 0110 deposited as CCAP number 1435/2 as a coating on a medical device for use in the preventive treatment of biofilm-mediated medical device associated infections in a subject.

2. The composition for use according to claim 1, wherein the medical device-associated infection is caused by *S. epidermidis* or *S. aureus,* preferably said medical device-associated infection is a catheter-associated infection.

3. The composition for use according to claim 1, wherein said composition is further used for preventing platelet adhesion to the composition and activation of said platelets.

4. A coated surface or article coated, wherein the coating is on a medical device and comprises the polymer released from *Cyanothece* sp. CCY 0110 deposited as CCAP number 1435/2, for use in the preventive treatment of biofilm-mediated medical device-associated infections in a subject.

5. The coated surface or article for use according to claim 4, wherein the biofilm is caused by *S. epidermidis* or *S. aureus.*

6. The coated surface or article for use according to claim 5, wherein the biofilm is caused by a highly adhesive *S. epidermidis* strain in the presence of human plasma proteins.

7. The coated surface or article for use according to claims 4 to 6, wherein said coated surface or article is further used for preventing platelet adhesion to the coated surface or article and activation of said platelets.

8. The coated surface or article coated for use according to claims 4-7 wherein said coated surface or article is implanted in an animal or human body.

9. The coated surface or article for use according to claim 8, wherein the coated surface or article further comprises at least one of the following components: a dye, a paint, a varnish, a polish, a primer, a surface activator, a coating or mixtures thereof.

10. The coated surface or article for use according to claim 8, wherein the coated surface or article further comprises at least 0.01% (w/v) of the polymer.

11. The coated surface or article for use according to claim 8, wherein the coated surface or article further comprises at least 0.05% (w/v) of the polymer, preferably wherein the coated surface or article further comprises at least 5% (w/v) of the polymer.

12. The coated surface or article for use according to claim 8, wherein the coated surface or article further comprises at least 10% (w/v) of the polymer, preferably wherein the coated surface or article further comprises at least 50% (w/v) of the polymer.

13. The coated surface or article for use according to claim 8, wherein the coated surface or article further comprises 0.01-2% (w/v) of polymer; preferably 0.05-1% (w/v) of polymer; more preferably 0.1-0.5% (w/v) of polymer.

14. The coated surface or article for use according to claims 8 to 13, wherein the coated surface or article is a medical device, preferably selected from the list consisting of a catheter, a syringe, a stent, a tube, a medical packaging, a dialysis device, a dental prothesis, dental implant, and an orthodontic device.

## Patentansprüche

1. Eine Zusammensetzung, umfassend das von Cyanothece sp. CCY 0110, hinterlegt bei der CCAP unter der Nummer 1435/2, freigesetzte Polymer, als eine Beschichtung auf einem medizinischen Gerät zur Verwendung bei der präventiven Behandlung von durch Biofilme verursachten, mit einem medizinischen Gerät assoziierten Infektionen bei einem Patienten.

2. Die Zusammensetzung zur Verwendung nach Anspruch 1, wobei die mit einem medizinischen Gerät verbundene Infektion durch *S. epidermidis* oder *S. aureus* verursacht wird, wobei die mit dem genannten medizinischen Gerät verbundene Infektion bevorzugt eine mit einem Katheter verbundene Infektion ist.

3. Die Zusammensetzung zur Verwendung nach Anspruch 1, wobei die genannte Zusammensetzung ferner zur Vorbeugung der Thrombozytenadhäsion an der Zusammensetzung und der Aktivierung der genannten Tyrombozyten verwendet wird.

4. Eine beschichtete Oberfläche oder ein beschichteter Gegenstand, wobei sich die Beschichtung auf einem medizinischen Gerät befindet und das von *Cyanothece* sp. CCY 0110, hinterlegt bei der CCAP unter der Nummer 1435/2, freigesetzte Polymer umfasst, für die Verwendung bei der präventiven Behandlung von durch Biofilme verursachten, mit einem medizinischen Gerät assoziierten Infektionen bei einem Patienten.

5. Die beschichtete Oberfläche oder der beschichtete Gegenstand zur Verwendung nach Anspruch 4, wobei der Biofilm durch *S. epidermidis* oder *S. aureus* verursacht wird.

6. Die beschichtete Oberfläche oder der beschichtete Gegenstand zur Verwendung nach Anspruch 5, wobei der Biofilm durch einen stark anhaftenden *S*. *epidermidis*-Stamm in Gegenwart menschlicher Plasmaproteine verursacht wird.

7. Die beschichtete Oberfläche oder der beschichtete Gegenstand zur Verwendung nach Ansprüche 4 bis 6, wobei die genannte beschichtete Oberfläche oder der genannte beschichtete Gegenstand ferner dazu verwendet wird, die Thrombozytenadhäsion an der beschichteten Oberfläche oder dem beschichteten Gegenstand und die Aktivierung der genannten Thrombozyten zu verhindern.

8. Die beschichtete Oberfläche oder der beschichtete Gegenstand zur Verwendung nach Ansprüche 4-7, wobei die genannte beschichtete Oberfläche oder der genannte beschichtete Gegenstand in einen tierischen oder menschlichen Körper implantiert wird.

9. Die beschichtete Oberfläche oder der beschichtete Gegenstand zur Verwendung nach Anspruch 8, wobei die beschichtete Oberfläche oder der beschichtete Gegenstand ferner mindestens eine der folgenden Komponenten umfasst: einen Farbstoff, eine Farbe, einen Lack, eine Politur, eine Grundierung, einen Oberflächenaktivator, eine Beschichtung oder Mischungen davon.

10. Die beschichtete Oberfläche oder der beschichtete Gegenstand zur Verwendung nach Anspruch 8, wobei die beschichtete Oberfläche oder der beschichtete Gegenstand ferner mindestens 0,01 % (w/v) des Polymers umfasst.

11. Die beschichtete Oberfläche oder der beschichtete Gegenstand zur Verwendung nach Anspruch 8, wobei die beschichtete Oberfläche oder der beschichtete Gegenstand ferner mindestens 0,05 % (w/v) des Polymers umfasst, wobei die beschichtete Oberfläche oder der beschichtete Gegenstand ferner bevorzugt mindestens 5 % (w/v) des Polymers umfasst.

12. Die beschichtete Oberfläche oder der beschichtete Gegenstand zur Verwendung nach Anspruch 8, wobei die beschichtete Oberfläche oder der beschichtete Gegenstand ferner mindestens 10 % (w/v) des Polymers umfasst, wobei die beschichtete Oberfläche oder der beschichtete Gegenstand ferner bevorzugt mindestens 50 % (w/v) des Polymers umfasst.

13. Die beschichtete Oberfläche oder der beschichtete Gegenstand zur Verwendung nach Anspruch 8, wobei die beschichtete Oberfläche oder der beschichtete Gegenstand ferner 0,01-2 % (w/v) Polymers; bevorzugt 0,05-1 % (w/v) Polymers; besonders bevorzugt 0,1-0,5 % (w/v) Polymers umfasst.

14. Die beschichtete Oberfläche oder der beschichtete Gegenstand zur Verwendung nach Ansprüche 8 bis 13, wobei die beschichtete Oberfläche oder der beschichtete Gegenstand ein medizinisches Gerät ist, bevorzugt ausgewählt aus der Liste bestehend aus einem Katheter, einer Spritze, einem Stent, einem Schlauch, einer medizinischen Verpackung, einer Dialysevorrichtung, einer Zahnprothese, einem Zahnimplantat und einer kieferorthopädischen Vorrichtung.

## Revendications

1. Une composition comprenant le polymère libéré à partir de Cyanothece sp. CCY 0110 déposé sous le numéro CCAP 1435/2 en tant que revêtement sur un dispositif médical destiné à être utilisé dans le traitement préventif d'infections associées à un dispositif médical utilisant un biofilm sur un patient.

2. La composition destinée à être utilisée selon la revendication 1, dans laquelle l'infection associée à un dispositif médical est causée par *S*. *epidermidis* ou *S*. *aureus,* l'infection associée au dispositif médical étant préférablement une infection associée à un cathéter.

3. La composition destinée à être utilisée selon la revendication 1, dans laquelle ladite composition est également utilisée pour prévenir l'adhésion de plaquettes à la composition et l'activation desdites plaquettes.

4. Une surface revêtue ou un article revêtu, dans lequel le revêtement est sur un dispositif médical et comprend le polymère libéré à partir de *Cyanothece* sp. CCY 0110 déposé sous le numéro CCAP 1435/2, destiné à être utilisé dans le traitement préventif d'infections associées à un dispositif médical utilisant un biofilm sur un patient.

5. La surface revêtue ou l'article revêtu destiné(e) à être utilisé(e) selon la revendication 4, dans lequel le biofilm est causé par *S*. *epidermidis* or *S*. *aureus.*

6. La surface revêtue ou l'article revêtu destiné(e) à être utilisé(e) selon la revendication 5, dans lequel le biofilm est causé par une souche de *S*. *epidermidis* hautement adhésive en présence de protéines plasmatiques humaines.

7. La surface revêtue ou l'article revêtu destiné(e) à être utilisé(e) selon les revendications 4 à 6, dans lequel ladite surface revêtue ou ledit article revêtu est également utilisé(e) pour prévenir l'adhésion de plaquettes à la surface revêtue ou à l'article revêtu et l'activation desdites plaquettes.

8. La surface revêtue ou l'article revêtu destiné(e) à être utilisé(e) selon les revendications 4-7 dans lequel ladite surface revêtue ou ledit article revêtu est implanté dans un animal ou un corps humain.

9. La surface revêtue ou l'article revêtu destiné(e) à être utilisé(e) selon la revendication 8, dans lequel la surface revêtue ou l'article revêtu comprend également au moins un des composants suivants : un colorant, une peinture, une laque, un vernis, un apprêt, un activateur de surface, un revêtement ou des mélanges de ceux-ci.

10. La surface revêtue ou l'article revêtu destiné(e) à être utilisé(e) selon la revendication 8, dans lequel la surface revêtue ou l'article revêtu comprend également au moins 0,01% (p/v) du polymère.

11. La surface revêtue ou l'article revêtu destiné(e) à être utilisé(e) selon la revendication 8, dans lequel la surface revêtue ou l'article revêtu comprend également au moins 0,05% (p/v) du polymère, préférablement dans lequel la surface revêtue ou l'article revêtu comprend également au moins 5% (p/v) du polymère.

12. La surface revêtue ou l'article revêtu destiné(e) à être utilisé(e) selon la revendication 8, dans lequel la surface revêtue ou l'article revêtu comprend également au moins 10% (p/v) du polymère, préférablement dans lequel la surface revêtue ou l'article revêtu comprend également au moins 50% (p/v) du polymère.

13. La surface revêtue ou l'article revêtu destiné(e) à être utilisé(e) selon la revendication 8, dans lequel la surface revêtue ou l'article revêtu comprend également 0,01- 2% (p/v) du polymère ; préférablement 0,05-1% (p/v) du polymère ; plus préférablement 0,1-0,5% (p/v) du polymère.

14. La surface revêtue ou l'article revêtu destiné(e) à être utilisé(e) selon les revendications 8 à 13, dans lequel la surface revêtue ou l'article revêtu est un dispositif médical, préférablement sélectionné à partir de la liste consistant en un cathéter, une seringue, un stent, un tube, un emballage médical, un dispositif de dialyse, une prothèse dentaire, un implant dentaire, et un dispositif orthodontique.
